(19) Europäisches Patentamt
European Patent Office
Office européen des brevets

(11) Veröffentlichungsnummer: **0 139 110**
**B1**

(12) **EUROPÄISCHE PATENTSCHRIFT**

(45) Veröffentlichungstag der Patentschrift: **03.06.87**

(51) Int. Cl.[4]: **C 08 G 59/68,** C 07 D 233/60, C 08 L 63/00

(21) Anmeldenummer: **84108723.2**

(22) Anmeldetag: **24.07.84**

# (54) Härtbare Epoxidharze.

(30) Priorität: **02.08.83 DE 3327823**

(43) Veröffentlichungstag der Anmeldung: **02.05.85 Patentblatt 85/18**

(45) Bekanntmachung des Hinweises auf die Patenterteilung: **03.06.87 Patentblatt 87/23**

(84) Benannte Vertragsstaaten: **CH DE FR GB IT LI**

(56) Entgegenhaltungen:
**DE - A - 3 246 072**
**GB - A - 2 066 697**

**Chemical Abstracts, Band 81, Nr. 16, 21 Oktober 1974, Columbus, Ohio, USA; Seite 43, Spalte 1, Abstract Nr. 92516W**

(73) Patentinhaber: **BASF Aktiengesellschaft, Carl-Bosch-Strasse 38, D-6700 Ludwigshafen (DE)**

(72) Erfinder: **Tesch, Helmut, Dr., Goethestrasse 12, D-6701 Birkenheide (DE)**
Erfinder: **Henne, Andreas, Dr., Adolf-Kolping-Strasse 137, D-6730 Neustadt (DE)**
Erfinder: **Heym, Manfred, Dr., Bachweg 6, D-6719 Weisenheim (DE)**
Erfinder: **Stutz, Herbert, Dr., Im Eichbaeumle 93, D-7500 Karlsruhe (DE)**

**EP 0 139 110 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die Erfindung betrifft Epoxidharze, die substituierte N-Acyl-Imidazole als Katalysatoren bzw. als Beschleuniger in Kombination mit üblichen Härtern enthalten.

Es ist bekannt, z.B. aus der DE-B-1 301 135, dass Imidazole mit Epoxidharzen bei erhöhter Temperatur sehr schnell zu Formstoffen mit guten physikalischen Eigenschaften reagieren. Die Imidazole initiieren eine anionische Polymerisation der Epoxidharze, wodurch ein dreidimensionales Netzwerk mit Polyether-Strukturen entsteht.

Weiterhin ist bekannt, z.B. aus der US-A-3 329 652, dass geringe Mengen Imidazole die Reaktion von Epoxidharzen mit Polycarbonsäureanhydriden als Härter beschleunigen können.

In beiden Fällen ist von Nachteil, dass die Mischungen bei Raumtemperatur und mässig erhöhter Temperatur nur geringe Zeit haltbar sind. Der Erfindung lag daher die Aufgabe zugrunde, eine härtbare Harzzusammensetzung auf Basis von Epoxidharzen zu entwickeln, die bei Raumtemperatur und mässig erhöhter Temperatur längere Zeit lagerstabil ist und erst bei starker Temperaturerhöhung schnell zu Formkörpern mit guten mechanischen Eigenschaften aushärtet.

Es wurde gefunden, dass diese Aufgabe gelöst wird, wenn man als Härter für Epoxidharze bzw. als Beschleuniger für übliche Härter enthaltende Epoxidharze N-Acyl-Imidazole mit einer aromatischen Acylkomponente, die an den beiden ortho-Positionen zur Carbonylgruppe substituiert ist, verwendet.

In der JP-A-74 007 599 werden N-Acyl-Imidazole mit einer unsubstituierten aromatischen Acylkomponente als Katalysatoren für Epoxidharze beschrieben. Diese Imidazole ergeben jedoch keine lagerstabilen Harzzusammensetzungen.

In der DE-A-32 46 072 sind N-Acyl-Imidazole beschrieben, bei denen die aromatische Acylgruppe halogen- oder nitrosubstituiert ist. Diese Imidazole sollen sich als Härter für Epoxidharze eignen. Es hat sich aber gezeigt, dass auch derartige Harzsysteme keine ausreichende Lagerstabilität aufweisen.

Gegenstand der Erfindung sind härtbare Harzzusammensetzungen, enthaltend

A. 100 Gew.-Teile eines Epoxidharzes,

B. 0,01 bis 50 Gew.-Teile eines N-Acyl-Imidazols der Formel I oder II nach Patentanspruch 1, sowie gegebenenfalls

C. 1 bis 200 Gew.-Teile eines üblichen Epoxidharzhärters.

«Epoxidharze» sind nieder- und hochmolekulare Verbindungen mit end- oder seitenständigen oder in cyclische Systeme eingebauten Epoxidgruppen. Sie können flüssig, halbfest oder fest sein. Bevorzugt sind Verbindungen, die mehr als eine Epoxidgruppe pro Molekül enthalten. Bevorzugte Epoxidharze sind Umsetzungsprodukte von mehrfach funktionellen Alkoholen, Phenolen, cycloaliphatischen Carbonsäuren, aromatischen Aminen oder Aminophenolen mit Epichlorhydrin, sowie cycloaliphatische Epoxide und cycloaliphatische Epoxidester. Es können auch Mischungen von verschiedenen Epoxidharzen zum Einsatz kommen. Besonders bevorzugt sind Bisphenol-A-Diglycidylether, Tetraglycidyldiaminodiphenylmethan und epoxidierte Novolake. Die Harze können die üblichen Verdünnungsmittel, wie Phenylglycidylether oder Butylglycidylether enthalten.

Die erfindungsgemäss einzusetzenden N-Acyl-Imidazole der Formel I und II sind bekannt. Ihre Herstellung ist z.B. in Liebigs Ann. Chem. 655, S. 90 (1962) und 694, Seite 78 (1966) beschrieben. Wenn sie als Katalysatoren für das Epoxidharz wirken sollen, werden sie bevorzugt in Mengen von 2 bis 20 Gew.-Teilen, bezogen auf 100 Gew.-Teile des Epoxidharzes, verwendet. Bei Bisphenol-A-Diglycidylether sind 5 bis 10 Gew.-Teile des N-Acyl-Imidazols besonders günstig. Gegebenenfalls können auch Mischungen der N-Acyl-Imidazole der Formeln I oder II untereinander oder mit anderen bekannten Epoxidkatalysatoren bzw. Beschleunigern eingesetzt werden.

Unter Epoxidharzhärtern im Sinne der Erfindung sind solche Härter zu verstehen, deren Reaktion mit dem Epoxidharz durch Zusatz eines nicht-acylierten Imidazols beschleunigt wird. Hierzu gehören Carbonsäuren, Carbonsäureanhydride, Carbonsäurehydrazide, Phenole, Benzoguanamin und Dicyandiamid. Bevorzugt sind die Anhydride der Phthalsäure, Tetrahydrophthalsäure, Methyltetrahydrophthalsäure, Hexahydrophthalsäure, Pyromellitsäure und Methylbicyclo[2.2.1]-hepten-2,3-dicarbonsäure, Isophthalsäuredihydrazid sowie Pyrogallol, Phloroglucin und insbesondere Dicyandiamid. Dicyandiamid ist in Epoxidharzen praktisch vollkommen unlöslich und deshalb für lagerstabile sog. «Einkomponentenharze» besonders geeignet.

Bevorzugte Zusammensetzungen enthalten auf 100 Gew.-Teile Epoxidharz 4 bis 100 Gew.-Teile des üblichen Härters und 0,1 bis 5 Gew.-Teile des N--Acyl-Imidazol-Beschleunigers. Bei Bisphenol-A-Diglycidylether sind 4 bis 10 Gew.-Teile Dicyandiamid und 0,2 bis 2 Gew.-Teile des N-Acyl-Imidazols bzw. 50 bis 100 Gew.-Teile eines Carbonsäureanhydrids und 0,1 bis 0,5 Gew.-Teile des N-Acyl-Imidazols besonders günstig.

Die härtbaren Zusammensetzungen können noch übliche Zusatzstoffe, wie Pigmente, Füllstoffe, Verstärkungsfasern, Flexibilisierungsmittel, Flammschutzmittel und nichtflüchtige Streckmittel enthalten.

Die erfindungsgemässen Epoxidharz-Zusammensetzungen werden mit Vorteil überall dort verwendet, wo eine gute Stabilität der noch nicht gehärteten Mischungen gefordert wird, beispielsweise bei Formgiessverfahren. Auch bei der Tränkung von faserigen Materialien, wo wegen der notwendigen niedrigen Viskosität der Harzmischung meist bei höherer Temperatur gearbeitet werden muss, ist eine lange Lagerfähigkeit des Tränkharzes erwünscht. Mischungen aus Epoxidharzen, Dicyandiamid und N-Acyl-Imidazolen der Formeln I oder II sind bei Raumtemperatur über mehrere Monate und bei 50 °C mehrere Tage stabil. Sie eignen sich hervorragend als Tränkharze für die Herstellung faserverstärkter Halbzeuge und Fertigteile. Derartige Faserverbundmaterialien enthalten im allgemeinen 30 bis 70 Vol.-% Glas-, Kohlenstoff- oder Aramid-Fasern in

Form von Endlosfasern, Bändern, Matten, Geweben oder Vliesen.

Enthalten die erfindungsgemässen Zusammensetzungen ein festes Epoxidharz, so können sie nach dem Reaktionsspritzgussverfahren verarbeitet werden. Man mischt die pulverförmigen Bestandteile, zusammen mit Füllstoffen oder Kurzfasern und Pigmenten, stellt durch Aufschmelzen eine homogene Masse her, lässt diese abkühlen und mahlt sie. Dabei ist es wesentlich, dass die Härtung bei der Schmelztemperatur, die in der Regel 50 bis 100°C beträgt, noch nicht in nennenswertem Masse einsetzt. Bei herkömmlichen Härtungsmitteln, die bei diesen Temperaturen schon anspringen, bleibt nur übrig, die Einzelbestandteile fein zu mahlen und die Pulver dann mechanisch zu vermischen. Dies ergibt jedoch keine ausreichende Homogenität.

Die erfindungsgemässen Epoxidharzzusammensetzungen werden durch Wärmezufuhr ausgehärtet. Die Härtungstemperatur richtet sich nach der Art des Epoxidharzes, des Härters und des N-Acyl-Imidazols. Sie kann in weiten Grenzen zwischen 50 und 300°C, vorzugsweise zwischen 80 und 200°C schwanken. Die bevorzugten Zusammensetzungen werden bei Temperaturen von 120 bis 160°C gehärtet.

Die erfindungsgemässen Zusammensetzungen können zur Herstellung von isolierenden Überzügen, Lacken, Einbettmassen, Klebstoffen oder für faserverstärkte Werkstoffe eingesetzt werden.

Die in den Beispielen genannten Prozente und Teile beziehen sich auf das Gewicht.

*Beispiele*

A. Herstellung der N-Acyl-Imidazole

N(2,4,6-Trimethylbenzoyl)-Imidazol

136 g (2,0 Mol) Imidazol werden in 1000 ml Ether vorgelegt und in 30 min bei 10 bis 20°C unter Rühren 146 g (0,8 Mol) 2,4,6-Trimethylbenzoylchlorid zugetropft. Es wird 5 h bei Raumtemperatur nachgerührt und das ausgefallene Imidazol-hydrochlorid abgesaugt. Das Filtrat wird eingeengt und destilliert. Dabei werden 116,4 g (68% d.Th.) N(2,4,6-Trimethylbenzoyl)-Imidazol, Sdp. 128°C/0,01 mbar, erhalten.

In analoger Weise wurden die anderen, in Tabelle 1 zusammengestellten, N-Acyl-Imidazole erhalten, wobei gegebenenfalls die Destillation durch einen Umkristallisationsschritt ersetzt wurde. Die Substanzen 7 bis 11 zählen nicht zu den erfindungsgemässen Härtungsmitteln. Sie wurden zu Vergleichszwecken hergestellt.

*Tabelle 1*

Folgende N-Acyl-Imidazole wurden hergestellt:

1 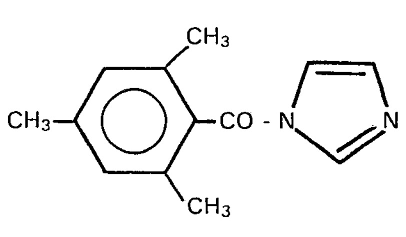

2 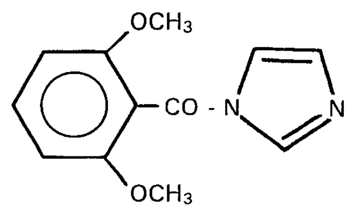

3 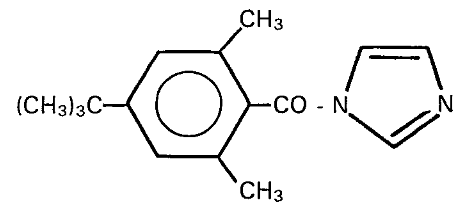

4 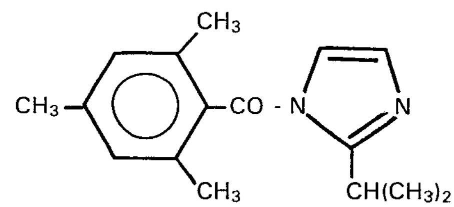

5 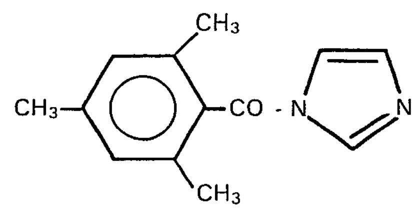

6 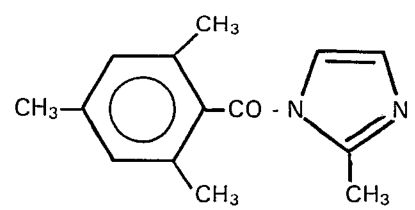

7 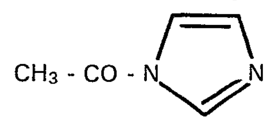

8 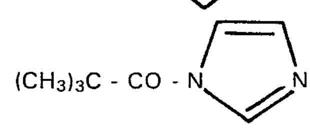

9 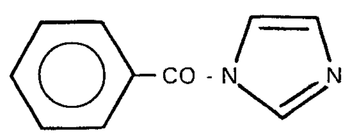

10 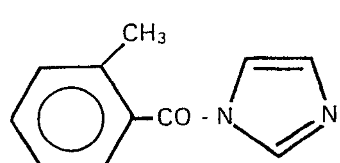

11 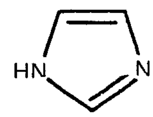

B. Härtungsversuche

Harzmischungen aus Bisphenol-A-diglycidylether (Epikote® 828 von SHELL) und dem N-Acyl-Imidazol werden durch Mahlen bei Raumtemperatur in einer Achatmühle hergestellt. Jeweils gleiche Mengen der Mischungen werden in Reagenzgläser gefüllt und in ein vorgeheiztes Ölbad gestellt. In die Proben taucht ein Thermoelement ein, wodurch der Verlauf der exothermen Härtungsreaktion verfolgt werden kann. Der Verlauf der Temperatur in Abhängigkeit der Zeit wird aufgezeichnet. Die charakteristischen Grössen der Kurve sind die Ansprechtemperatur (= Badtemperatur), die Zeit von Angleichung der Probentemperatur an die Badtemperatur bis zum Erreichen der Maximaltemperatur, sowie die Maximaltemperatur selbst.

Zur Überprüfung der Lagerstabilität der Mischungen bei Raumtemperatur werden die Viskositäten der Mischungen in Abhängigkeit der Lagerzeit in einem Rotationsviskosimeter (Rheomat 15 T von Contraves) gemessen.

Die Ergebnisse der Versuche sind in Tabelle 2 zusammengestellt. Dabei sind auch Vergleichsbeispiele mit den nicht erfindungsgemässen Imidazolen A7 bis A11 aufgenommen.

Die Konzentration ist in Teilen Imidazol pro 100 Teile Epoxidharz angegeben, die Viskosität der Mischungen bei 23°C in [mPas] wurde sofort nach Herstellung der Mischung bzw. nach dreitägiger Lagerung gemessen.

*Tabelle 2*

| Katalysator | Konz. | Ansprech-temp. (°C) | Härtungs-zeit [min] | Maximal temp. (°C) | Viskosität [mPas] sofort | nach 3 Tg. |
|---|---|---|---|---|---|---|
| A 1 | 6,3 | 120° | 7,0 | 217 | 10.000 | 16.000 |
| A 2 | 6,8 | 120° | 7,7 | 243 | 30.000 | 38.000 (2Tg) |
| A 3 | 7,5 | 120° | 11,2 | 249 | 21.000 | 31.000 (2Tg) |
| A 4 | 9,0 | 160° | 12,0 | 193 | 6.000 | 12.000 |
| A 5 | 10,0 | 140° | 10,5 | 245 | | |
| A 6 | 6,7 | 120° | 6,6 | 216 | | |
| A 7 | 2,9 | 120° | 2,7 | 233 | 13.000 | $10^6$ |
| A 8 | 4,5 | 120° | 2,9 | 241 | 13.000 | $10^6$ |
| A 9 | 4,7 | 120° | 2,8 | 238 | 15.000 | $10^6$ |
| A10 | 5,5 | 120° | 2,8 | 262 | 16.000 | $10^6$ |
| A11 | 2,0 | 120° | 1,5 | 236 | 22.000 | $10^6$ |

C. Analog den in B beschriebenen Beispielen wurden folgende Mischungen hergestellt:

1. 50,0 Teile Epikote 828
   44,5 Teile Methyltetrahydrophthalsäureanhydrid
2. 50,0 Teile Epikote 828
   44,5 Teile Methyltetrahydrophthalsäureanhydrid
   0,25 Teile N-Acyl-Imidazol A1
3. 50,0 Teile Epikote 828
   11,5 Teile Pyrogallol
4. 50,0 Teile Epikote 828
   11,5 Teile Pyrogallol
   0,25 Teile N-Acyl-Imidazol A1

Die Versuche C1 und C3 enthalten nur übliche Härter ohne das erfindungsgemässe N-Acyl-Imidazol als Beschleuniger. Tabelle 3 zeigt die Ergebnisse.

*Tabelle 3*

| Ansatz | Ansprech-temp. | Härtungs-zeit | Maximal-temp. |
|---|---|---|---|
| C1 | 120° | 90 min | — |
| C2 | 120° | 19,5 min | 133° |
| C3 | 160° | 90 min | — |
| C4 | 160° | 1,8 min | 237° |

D. Dieses Beispiel erläutert die Herstellung sowie die Aushärtung eines unidirektional mit Glasfasern verstärkten Epoxidharzlaminats mit Dicyandiamid als Härter und dem N-Acyl--Imidazol A1 als Beschleuniger.

62 Teile Epikote 828, 30 Teile epoxidierter Novolak (Eposid 5055 von Duroplast Chemie), 8 Teile Dicyandiamid und 0,5 Teile N-Acyl-Imidazol A1 werden bei 80°C homogenisiert. Diese Mischung hat bei 40°C eine Viskosität von 2700 mPas und ist bei Raumtemperatur mehrere Monate lagerstabil.

Zur Herstellung der Laminate wurde die Mischung in einem Tränkbad zunächst auf 80 bis 95°C erwärmt, um eine für die Tränkung der Glasfasern ausreichend niedrige Viskosität von 500 bis 1000 mPas einzustellen. Danach wurde ein Glasroving mit einem Titer von 1200 tex (EC 14-P 185≥1200 von GEVETEX) durch das Tränkbad gezogen und anschliessend so auf einer Trommel abgelegt, dass die getränkten Rovings parallel nebeneinander lagen. Nach Aufschneiden der Wickelung senkrecht zur Faserrichtung erhielt man ein einlagiges Prepreg mit unidirektionaler Faseranordnung und einem Fasergehalt von ca. 40 Vol.-%. Acht solcher Prepreglagen mit den Abmessungen 250×400 mm wurden mit gleicher Faserorientierung übereinandergeschichtet und in ein auf 80°C vorgeheiztes Plattenpresswerkzeug mit 2 mm Formspalt eingelegt. Danach wurde das Werkzeug innerhalb 15 min auf Anschlag geschlos-

sen, die Werkzeugtemperatur auf 150°C erhöht und der Prepregstapel innerhalb 30 min zum Laminat ausgehärtet. Nach Abkühlung und Entformung wurde das Laminat 60 min bei 170°C nachgehärtet.

Der Glasgehalt des Laminats betrug 65 Gew.-%. Die thermischen und mechanischen Eigenschaften sind nachfolgend zusammengestellt.

| | |
|---|---|
| Zugfestigkeit parallel zur Faserrichtung | 1020 $N/mm^2$ |
| Zug-Elastizitätsmodul parallel zur Faserrichtung | 33800 $N/mm^2$ |
| Bruchdehnung parallel zur Faserrichtung im Zugversuch | 3,0 % |
| Zugfestigkeit senkrecht zur Faserrichtung | 64 $N/mm^2$ |
| Zug-Elastizitätsmodul senkrecht zur Faserrichtung | 11300 $N/mm^2$ |
| Bruchdehnung senkrecht zur Faserrichtung im Zugversuch | 0,7 % |
| Glasübergangstemperatur (Torsionsschwinganalyse) | 150°C |

**Patentansprüche**

1. Härtbare Harzzusammensetzung, enthaltend

A. 100 Gew.-Teile eines Epoxidharzes,

B. 0,01 bis 50 Gew.-Teile eines N-Acyl-Imidazols, sowie gegebenenfalls

C. 1 bis 200 Gew.-Teile eines üblichen Epoxidharzhärters,

dadurch gekennzeichnet, dass Komponente B ein N-Acyl-Imidazol der Formeln

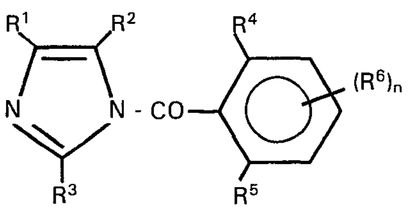

I

oder

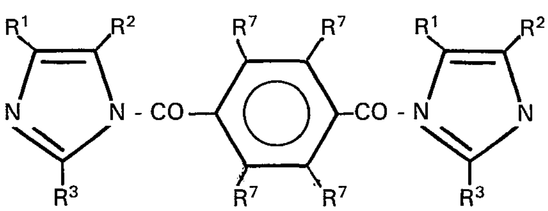

II

ist, wobei die Substituenten folgende Bedeutung haben:

$R^1$, $R^2$ und $R^3$ können gleich oder verschieden sein und für Wasserstoff, eine verzweigte oder unverzweigte Alkylgruppe mit 1 bis 17 Kohlenstoffatomen, ein Chloratom, einen Phenylrest, der gegebenenfalls durch Alkyl, Alkoxy oder Chlor substituiert ist, eine Halogenalkyl-, Hydroxyalkyl-, Carbonsäureester- oder Carbonsäureamid-Gruppe stehen;

$R^4$ und $R^5$ können gleich oder verschieden sein und für eine verzweigte oder unverzweigte Alkyl-, Alkoxy- oder Alkylthio-Gruppe mit 1 bis 4 Kohlenstoffatomen stehen;

$R^6$ kann für ein Wasserstoffatom, eine Alkoxy- oder Alkylthiogruppe mit 1 bis 6 Kohlenstoffatomen, eine Dialkylaminogruppe mit Alkylgruppen mit 1 bis 6 Kohlenstoffatomen oder eine verzweigte oder unverzweigte Alkylgruppe mit 1 bis 12 Kohlenstoffatomen stehen;

n kann für die Zahlen 1, 2 oder 3 stehen;

$R^7$ kann für eine Alkylgruppe mit 1 bis 4 Kohlenstoffatomen stehen.

2. Härtbare Harzzusammensetzung nach Anspruch 1, dadurch gekennzeichnet, dass sie

A. 100 Gew.-Teile eines Epoxidharzes und

B. 2 bis 20 Gew.-Teile des N-Acyl-Imidazols als Katalysator enthält.

3. Härtbare Zusammensetzung nach Anspruch 1, dadurch gekennzeichnet, dass sie

A. 100 Gew.-Teile eines Epoxidharzes,

B. 0,1 bis 5 Gew.-Teile des N-Acyl-Imidazols als Beschleuniger und

C. 4 bis 100 Gew.-Teile eines üblichen Epoxidharzhärters enthält.

4. Härtbare Zusammensetzung nach Anspruch 3, dadurch gekennzeichnet, dass sie

A. 100 Gew.-Teile des Bisphenol-A-Diglycidylethers,

B. 0,2 bis 2 Gew.-Teile des N-Acyl-Imidazols und

C. 4 bis 10 Gew.-Teile Dicyandiamid enthält.

5. Verfahren zur Herstellung von Formteilen durch Einbringen der härtbaren Zusammensetzung nach Anspruch 1 in eine Form und Aushärten bei Temperaturen zwischen 50 und 300°C.

6. Verfahren zur Herstellung von Lacken, Überzügen und Klebstoffen durch Aufbringen der härtbaren Zusammensetzungen nach Anspruch 1 auf ein Substrat und Aushärten bei Temperaturen zwischen 50 und 300°C.

7. Verfahren zur Herstellung von Faserverbundwerkstoffen durch Tränken von 30 bis 70 Vol.-% Verstärkungsfasern mit 70 bis 30 Vol.-% der härtbaren Zusammensetzung nach Anspruch 1 und Aushärten bei Temperaturen zwischen 50 und 300°C.

**Claims**

1. A curable resin composition containing

A. 100 parts by weight of an epoxy resin,

B. from 0.01 to 50 parts by weight of a N-acylimidazole, and, if desired,

C. from 1 to 200 parts by weight of a conventional curing agent for epoxy resins,

wherein component B is an N-acylimidazole of the formula

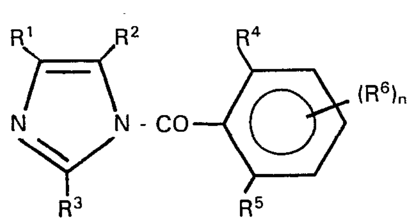

I

or

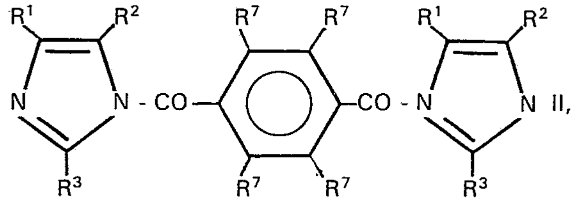 II,

where the substituents have the following meanings:

$R^1$, $R^2$ and $R^3$ can be identical or different and are each hydrogen, branched or straight-chain alkyl of 1 to 17 carbon atoms, chlorine, or phenyl which is unsubstituted or substituted by alkyl, alkoxy or chlorine, or are each haloalkyl, hydroxyalkyl, a carboxylic ester group or a carboxylamide group;

$R^4$ and $R^5$ can be identical or different and are each a branched or straight-chain alkyl, alkoxy or alkylthio group of 1 to 4 carbon atoms;

$R^6$ is hydrogen, an alkoxy or alkylthio group of 1 to 6 carbon atoms, dialkylamino where alkyl is of 1 to 6 carbon atoms, or branched or straight-chain alkyl of 1 to 12 carbon atoms;

n is 1, 2 or 3; and

$R^7$ is alkyl of 1 to 4 carbon atoms.

2. A curable composition as claimed in claim 1, which contains

A. 100 parts by weight of an epoxy resin, and

B. from 2 to 20 parts by weight of an N-acylimidazole as catalyst.

3. A curable composition as claimed in claim 1, which contains

A. 100 parts by weight of an epoxy resin,

B. from 0.1 to 5 parts by weight of an N-acylimidazole as accelerator, and

C. from 4 to 100 parts by weight of a conventional curing agent for epoxy resins.

4. A curable composition as claimed in claim 3, which contains

A. 100 parts by weight of bisphenol A diglycidyl ether,

B. from 0.2 to 2 parts by weight of an N-acylimidazole, and

C. from 4 to 10 parts by weight of dicyanodiamide.

5. A process for the production of a molding by introducing a curable composition as claimed in claim 1 into a mold, and carrying out curing at from 50 to 300°C.

6. A process for the production of a finish, a coating or an adhesive by applying a curable composition as claimed in claim 1 to a substrate, and carrying out curing at from 50 to 300°C.

7. A process for the production of a composite fiber material by impregnating from 30 to 70 vol.-% of reinforcing fibers with from 70 to 30 vol.-% of a curable composition as claimed in claim 1, and carrying out curing at from 50 to 300°C.

**Revendications**

1. Composition de résine durcissable, contenant

A. 100 parties en poids d'une résine époxyde;

B. 0,01 à 50 parties en poids d'un N-acylimidazole, ainsi qu'éventuellement

C. 1 à 200 parties en poids d'un durcisseur de résine époxyde usuel

caractérisée par le fait que le composant B est un N-acylimidazole de formule

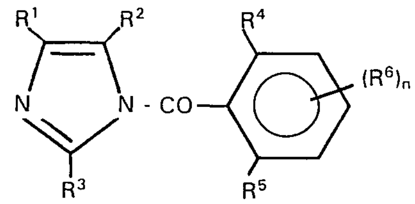 I

ou

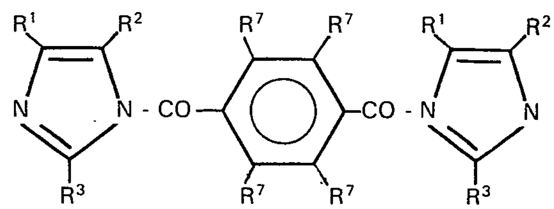 II

dans lesquelles les substituants ont les significations suivantes:

$R^1$, $R^2$ et $R^3$ peuvent être identiques ou différents et représenter l'hydrogène, un groupe alkyle ramifié ou non ramifié, à 1 à 17 atomes de carbone, un atome de chlore, un reste phényle éventuellement substitué par alkyle, alcoxy, ou chlore, un groupe halogénalkyle, hydroxyalkyle, ester ou amide d'acide carboxylique;

$R^4$ et $R^5$ peuvent être identiques ou différents et représenter un groupe alkyle, alcoxy ou alkylthio, ayant 1 à 4 atomes de carbone, ramifié ou non ramifié;

$R^6$ peut représenter un atome d'hydrogène, un groupe alcoxy ou alkylthio ayant 1 à 6 atomes de carbone, un groupe dialkylamino à groupes alkyle de 1 à 6 atomes de carbone ou un groupe alkyle ramifié ou non ramifié ayant 1 à 12 atomes de carbone;

n peut représenter les chiffres 1, 2 ou 3;

$R^7$ peut représenter un groupe alkyle de 1 à 4 atomes de carbone.

2. Composition de résine durcissable selon la revendication 1, caractérisée par le fait qu'elle contient

A. 100 parties en poids d'une résine époxyde et

B. 2 à 20 parties en poids de N-acylimidazole, comme catalyseur.

3. Composition de résine durcissable selon la revendication 1, caractérisée par le fait qu'elle contient

A. 100 parties en poids d'une résine époxyde,

B. 0,1 à 5 parties en poids de N-acylimidazole, comme accélérateur, et

C. 4 à 100 parties en poids d'un durcisseur de résine époxyde usuel.

4. Composition de résine durcissable selon la revendication 3, caractérisée par le fait qu'elle contient

A. 100 parties en poids d'éther bisphénol-A-diglycidylique,

B. 0,2 à 2 parties en poids de N-acylimidazole et

C. 4 à 10 parties en poids de dicyanodiamide.

5. Procédé de fabrication de pièces moulées par introduction de la composition durcissable selon la revendication 1 dans un moule et durcissement à des températures comprises entre 50 et 300°C.

6. Procédé de préparation de laques, enduits et colles par application de la composition durcissable selon la revendication 1 sur un substrat et durcissement à des températures comprises entre 50 et 300°C.

7. Procédé de fabrication de matériaux composites fibreux par imprégnation de 30 à 70% en volume de fibres de renforcement avec 70 à 30% en volume de la composition durcissable selon la revendication 1 et durcissement à des températures comprises entre 50 et 300°C.